Europäisches Patentamt

**European Patent Office**

**Office européen des brevets**

(19)

(11) Publication number: **0 128 527**
**A2**

(12) # EUROPEAN PATENT APPLICATION

(21) Application number: **84106468.6**

(22) Date of filing: **06.06.84**

(51) Int. Cl.³: **C 12 Q 1/18, C 12 Q 1/04**

(30) Priority: **09.06.83 US 502678**

(43) Date of publication of application: **19.12.84**
**Bulletin 84/51**

(84) Designated Contracting States: **CH DE FR IT LI NL SE**

(71) Applicant: **Murray, Marvin, 734 Fairhill Drive, Louisville Kentucky 40207 (US)**

(72) Inventor: **Murray, Marvin, 734 Fairhill Drive, Louisville Kentucky 40207 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) Accelerated determination of antimicrobial susceptibility of bacteria.

(57) A method is provided for accelerated determination of antimicrobial susceptibility of oxiphilic bacteria. The method utilizes the measurement of oxygen partial pressure ($pO_2$), and conditions for promoting rapid growth of the bacteria with a minimal lag growth phase. The $pO_2$ measurements are made in the liquid phase of the media towards the end of the lag phase or near the beginning of the logarithmic growth phase. The method is preferably carried out in a specially designed microdilution plate providing small volume spherical wells.

EP 0 128 527 A2

ACCELERATED DETERMINATION OF

ANTIMICROBIAL SUSCEPTIBILITY OF BACTERIA

FIELD OF INVENTION AND BACKGROUND

The field of this invention is that of methods and apparatus for antimicrobial susceptibility testing. The invention is particularly concerned with rapid procedures in which the minimal inhibitory concentration (MIC) of the antibiotic or other antimicrobial can be determined in a much shorter time than the standard microdilution or agar diffusion procedures.

The agar diffusion method of determining the susceptibility of a bacterium to a particular antibiotic is commonly used to obtain semi-quantitative readings. For example, using the standard Kirby-Bauer procedure, zones of inhibition on agar plates can be interpreted as showing that the bacterium is "susceptible" or "resistant". An incubation time of 16 to 18 hours is commonly employed.

More quantitative results are obtained by microdilution test procedures. A series of samples are prepared in microdilution trays containing the bacterium at a standard concentration and progressive dilutions of the antibiotic being tested. In the standard procedure for determining the MIC endpoint, samples are incubated for 16 to 18 hours, after which the extent of bacterial growth or inhibition is determined by measuring the turbidity of the samples. The endpoint is taken as the lowest concentration of the antimicrobic at which the microorganism tested does not show growth. The criteria of growth is a definite turbidity a single sedimented cluster (button) of organisms 2 mm or greater in diameter, or more than one cluster (even if less than 2 mm in diameter.

The need for a more rapid method of determining the minimal inhibitory concentration (MIC) has been recognized, and attempts have been made to develop mechanized and automated systems for yielding rapid and reliable results. One such system which has been proposed utilizes differential light scattering (DLS). A monochromatic laser beam is used to illuminate the samples prepared in appropriate dilution. The light scattering patterns produced are sensitive to changes in scattering particles. Susceptible bacteria in the presence of an antibiotic should have a scatter pattern that significantly differs from the control suspension (no antibiotic). However, it has not been demonstrated that the DLS system can be used for making reliable determinations of antimicrobial susceptibility.

Another type of system is known as the Rapid Sequential Bacteriological Analyzer (RSBA). This system is based on the use of sequential turbidometric microbial growth rate measurements. The system has been proposed for both qualitative and quantitative antimicrobial susceptibility tests. The results are to be calculated and displayed as MIC values. However, this system is complicated and expensive. Further, it has not yet been fully evaluated, or demonstrated as reliable for routine clinical laboratory use.

Most so-called "rapid systems" for susceptibility testing have had as their object the determination of MIC's in about three hours. Such short-time results compare with the conventional long-time result of 18-24 hours by either diffusion or microdilution tests. There has been a problem in demonstrating reliable correlation between such short term MIC's and the standard long term MIC's. Since the 18-hour MIC's represent the present reference indexes, it is desired to provide a short term procedure where the data can be correlated with the standard long term results. The unrealized objective is not only to provide such correlation, but also to reliably determine MIC's in much shorter times than three hours. Heretofore, no procedure for doing so has been established.

## SUMMARY OF INVENTION

The present invention utilizes a principle that has not heretofore been proposed or used for antimicrobial susceptibility testing. In the method of this invention, oxygen partial pressures ($pO_2$) in the liquid media are employed as the criteria for determining minimum inhibatory concentrations on an accelerated basis. Apparatus for measuring oxygen partial pressures in aqueous media, such as blood and serum, are available and in commercial use. However, such apparatus has not heretofore been applied to the testing of the action of antibiotics against bacteria.

For use of the method, the bacteria must be oxygen-consuming (viz. oxiphilic). However, most common pathogens are either aerobic or micro-aerophilic bacteria, both classes of which utilize oxygen from the media. In prior art microdilution test procedures, the liquid media contains oxygen which is utilized by the growing bacterium, and additional oxygen from the air diffuses into the medium through the liquid air interface. It may be assumed where bacteria growth has occurred that at the end of the conventional 18-hour incubation the oxygen content of the liquid media will be lower than at the beginning of the incubation. However, as far as is known, there is no published data with respect to such measurement. Further, there is no known theoretical basis for correlating the extent of oxygen depletion in the media of the microdilution test plates with standard MIC's.

However, the method of this invention does find scientific support in studies of bacterial growth characteristics published in the early 1930's. Martin, published observations that the oxygen consumption of aerobic bacteria (coli) reached a maximum on an $O_2$ per cell basis near the end of the lag phase. J. Gen. Physiol., 15,691 (1932). Hershey et al plotted values calculated from the data of Martin, which indicate that $O_2$ consumption per cell per minute generally follows the growth rate, and that the rate of cell division and the rate of oxygen consumption tend to reach maximums around the onset of the logarithimic growth phase. Proc. Soc. Exp. Biol. & Med., 36, 556 (1937). Other related studies published around the same time are: Eaton, J. Bact. 21, 143 (1931); and Gerard et al, Biol. Bull., 60, 213 (1931). In these studies, oxygen consumption was measured manometrically, the manometer being connected to the air space above the samples contained in closed relatively large volume flasks. In a later publication, Hershey et al found that Bacterium coli when just entering the phase of logarithimic multiplication consume oxygen at six times the rate pervailing near the end of the growth phase. J. Gen. Phys.,21, 721 (1938).

The method of this invention in preferred embodiments utilizes measurements of oxygen partial pressure in the liquid phase of the media of the microdilution test wells during the latter part of the lag phase or the early part of the logarithimic growth phase. For example, the $pO_2$ may be measured with an oxygen electrode, immediately before or immediately after the onset of logarithimic growth. In optimized embodiments, culturing is carried out using a medium and other culture conditions which reduce the length of the lag phase to a minimum, such as from 3 to 20 minutes. A "hyping-type" medium is preferably employed. Further, the test samples are preferably of small volume (0.5 to 1.5 ml) and contain a high concentration of the bacterium

$(10^6$ to $10^8)$. In a specific preferred embodiment, the samples are cultured in wells having generally spherical walls. A specifically designed microdilution plate is therefore provided for this purpose.

REFERENCE TO DRAWINGS

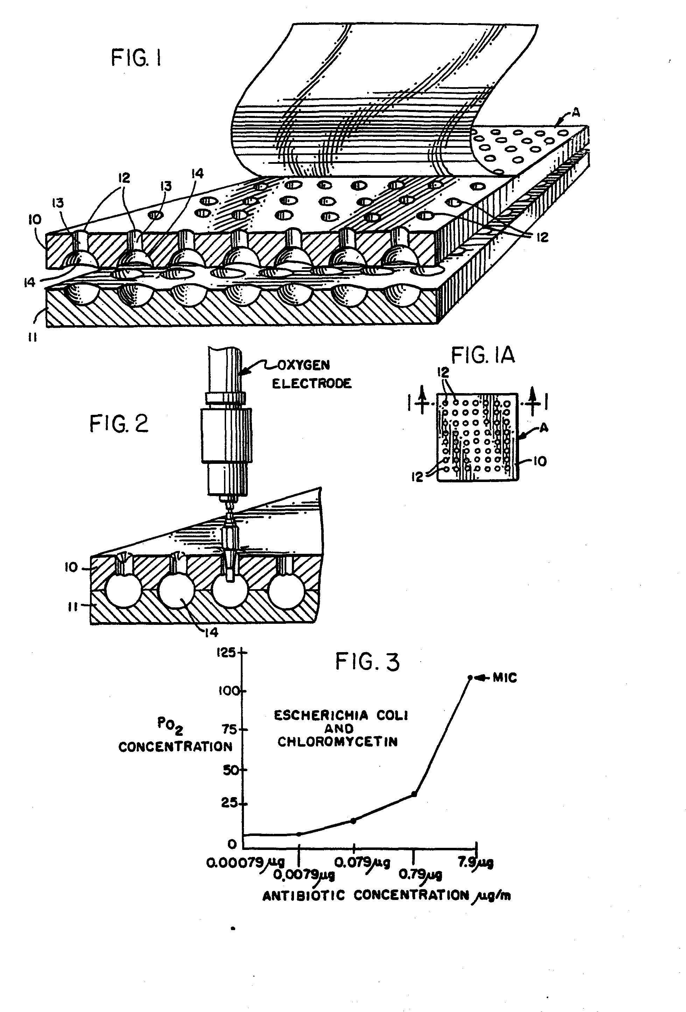

The accompanying drawing illustrates certain aspects of the present invention. Fig. 1 is a perspective of a microdilution test plate of preferred design, the components of the assembley being shown in partially separated relation, and one of the transverse rows of wells being shown in section.

Fig. 1A is a reduced scale top plan view of the plate assembley of Fig. 1 showing the arrangement of the wells.

Fig. 2 is a fragmentary partially sectioned perspective view of the assembley of Fig. 1 showing the media within the spherical wells and illustrating the insertion of an oxygen electrode probe through the plastic film covering the access ports.

Fig. 3 is a graph illustrating a representative set of measurements of $pO_2$ concentration versus antibiotic concentration showing the MIC point.

DETAILED DESCRIPTION

In one of its major aspects, this invention provides a method for accelerated determination of antimicrobial susceptibility of oxiphilic bacteria. Typically, the bacteria will be pathogens, classified as aerobic or micro-aerophilic. Within the broad class of oxiphilic pathogens the method is believed to be generally applicable.

As with the present procedures for determining antibiotic susceptibility, a pure culture of the bacteria to be tested must first be obtained. This is done by standard methods of isolation, as described for example in the Manual of Clinical Microbiology, Lennette, Editor, 3rd ed. 1980, 68-71.

Culture media as used in microdilution test procedures may be employed. However, it is preferred to utilize hyping-type media, which are designed to promote especially rapid growth. For example, a Brain Heart Infusion Medium may be employed. Such media are available commercially (viz. "DIFCO"). Growth promoting additives may be used, such as sugars metabolized by the microorganism. Such sugars, for example, include lactose, gelactose, sucrose, and dextrose, which may be used together. For example, in a specific embodiment, 0.5 grams of each of these four sugars are dissolved per liter of a Brain Heart Medium. The prepared media should be sterilized, such as by autoclaving.

A series of sterile samples of the aqueous culture medium are prepared. Usually at least each of positive and negative control samples will be included. The samples should be of corresponding volume and composition and should have a known concentration of dissolved oxygen. A conventional microdilution plate may be utilized to provide wells for the samples. It is preferred, however, that the wells of the plate have walls of a generally spherical configuration. The walls of conventional plates are generally cylindrical rather than spherical. Further, it is preferred to provide a minimum interface between the liquid of the sample and the air space above the sample. These features of the microdilution plate can be understood more clearly by reference to Figs. 1 and 2 of the accompanying drawing. In Fig. 1, the components of the plate assembley are shown in separated relation. In Fig. 2 the assembley is shown in unitary form, as used for the test procedure.

The plate assembley is formed from two superposed plate sections 10 and 11. The assembley is designated generally by the letter A. The upper section 10 provides a plurality of tranversely and longitudinally aligned access ports 12. In the illustration given, as shown more clearly in Fig. 1A there are seven ports in the transverse rows, and eight ports in the longitudinal rows. The ports 12 communicate with tubular necks 13, which connect to hemispherical recesses 14. As will be noted, the recesses are of enlarged diameter relative to the diameters of the port and neck portions.

The lower plate section 11 provides complementary hemispherical recesses 15, which in the assembled plate, as shown in Fig. 2, provide generally spherical cavities for receiving the oxygen-containing cultures. As shown in partially pulled-back relation in Fig. 1, a cover film 16 may be provided for sealing the ports 12 after introducing the test cultures.

Preferably plate sections 10 and 11 are formed of clear thermoplastic, and the plates are manufactured by injection molding or other suitable molding procedure. For example, the plates may be formed of an acrylic resin such as polymethylmethacrylate. The plates are preferably united along the center line of the recesses 14, 15 by either thermoplastic fusion or by the use of a suitable resin adhesive such as a silicone adhesive. The cover film 16 is also preferably formed from a clear plastic film, such as films formed from acrylic, polyolefin, saran, etc. A pressure-sensitive adhesive coating may be provided on the underside of the film 16. For example, the adhesive coated film sold as No. 853 by Minnesota Mining & Manufacturing Company may be employed, or other similar films coated with a pressure-sensitive adhesive on one side.

The preferred volume of the semicircular cavities of the plate (not including the volume of the neck portion 13) is from about 0.5 to 1.5 milliliters (ml). For example, to provide a spherical volume of about 1 ml, cavities may have a diameter of 1.24 cm. Correspondingly, the neck portion 13 may have a height of 0.5 centimeters and a diameter of 0.5 cm. The diameter of the ports 12 and the necks 13 should be sufficient to permit the insertion of the probe of an oxygen electrode.

After the cavities formed by the recesses 14 and 15 have been filled with the culture samples, the film 16 is applied to close the ports 12. As illustrated in Fig. 2, it is not necessary to remove the film for reading the oxygen partial pressures. As there shown, the two left hand ports 12 have already been opened by punching through the film over the openings with the oxygen electrode, and the sensing probe 17 of the oxygen electrode 18 is shown inserted in the third port, while the fourth port remains closed by the sealing film.

In a preferred use of the plate assembley A, the liquid medium of the culture samples is filled approximately to the bottom of the necks 13, or slightly into the lower portion of the necks, the desired location of the fill line being indicated in Fig. 2. It is desired to reduce the interface between the liquid medium and the air space there-above in the necks 13 to a relatively small area, thereby tending to minimize the transfer of oxygen from the neck air spaces into the liquid medium.

The oxygen electrode may be of the kind described by Clark, _Trans. Am. Soc. Artif. Intern. Organs_, 2, 41 (1956). A commercial version of the Clark electrode is available from Corning Medical, Medfield, Mass. The Corning oxygen electrode may be used as a component of its 175 Automatic p/A Blood Gas System. In general, suitable oxygen electrodes consist of a platinum cathode, and Ag/AgCl anode, an electrolite filling solution, and a polypropylene membrane. The circuit is completed at the anode, where silver is oxidized. The magnitude of the resulting current indicates partial pressure of the oxygen in the sample.

Certain of the steps of the method of this invention can be carried out by similar or identical procedures to those used for standard microdilution testing. The techniques for preparing the series of sterile samples of the culture medium, the incorporation of the water-soluble antimicrobial in the samples with selected progressive dilutions, the inoculating of the samples with predetermined quantities of the culture to obtain a selected cell concentration, and the culturing of the samples may, in general, be carried out by substantially identical procedures to the standard microdilution antimicrobial susceptibility tests.

Where the sample series includes positive and negative control samples, as preferred, the positive control is inoculated with the bacteria while the negative control is not inoculated. Both control samples are kept free of the antibiotic. As previously explained, it is preferred to employ a culture medium which is formulated to promote rapid growth of the test bacterium, such as a hyping-type medium. Further, it is desirable to incorporate a relatively high concentration of the bacterium in the samples, such as a concentration in the range of $10^6$ to $10^8$ cells/ml. The microdilution procedure usually involves cell concentrations

of about $10^6$ cells/ml. For the purpose of this invention, it is believed that somewhat higher concentrations are desirable, such as $10^7$ to $10^8$ cells/ml. The objective is short term culturing with the measurements being made before much cell division has occurred, such as in the periods from 5 minutes before to 40 minutes after the start of the incubation. In the latter part of the lag phase, if the growth of the cells has not been inhibited by the antibiotic, with either bactericidal or bacteriostatic action, the non-inhibited cells will be using oxygen at a high rate, which will in turn reduce the oxygen concentration of the medium, which reduction can be read in terms of reduced partial pressure ($pO_2$). In preferred embodiments, the readings can be made during the transitional period comprising the end of the lag phase and the beginning of the growth phase.

This procedure of this invention can provide greatly accelerated determinations of antibiotic susceptibility. However, if desired, meaningful readings of oxygen partial pressure reduction can also be made at a later stage in culturing, such as at an intermediate time in the logarithimic growth phase. For example, determinations of oxygen partial pressure reduction can be made in the period of 3 to 4 hours after the start of the incubation. Such readings also provide accelerated determinations as compared with the standard incubations of 16 to 18 hours.

Fig. 3 of the drawing is a plot of the partial pressure readings obtained in application of the method of this invention to the testing of E. coli for susceptibility to chloromycetin. All readings were taken at 3.5 hours. The medium was Brain Heart Infusion broth containing per liter of medium 0.5 grams each of lactose, galactose, sucrose, and dextrose. The medium was sterilized by autoclaving prior to inoculation, and the incubations were carried out at 37°C in 500 ml flasks containing 125 ml of broth. The concentration of the antibiotics in the 5 flasks containing the antibiotic are indicated on the horizontal access of Fig. 3 in micrograms per milliliter ($\mu$g/ml). The scale for the oxygen partial pressures (p$O_2$) is shown on the vertical axis. The oxygen partial pressure in the two control flasks containing only the broth was 144-146. In the control flask containing the broth and bacteria without the antibiotic, at the end of 3.5 hours the p$O_2$ was reduced to 7.2. The antibiotic concentration of 0.79 $\mu$g showed some inhibition, while inhibition approaching the non-inoculated controls was at 7.9 $\mu$g. This value was therefore read as the minimum inhibitory concentration (MIC). The corresponding value as determined by standard microdilution test is about 8.0 $\mu$g. See comparative data collected in the Manual of Clinical Microbiology, Lennette, Editor, 3rd ed. 1980, Appen. 2, 498-499.

Techniques of using the method of this invention with the plate assembley of Figs. 1 and 2 are more fully illustrated by the following examples.

EXAMPLES

Using cassette plates like that of Figs. 1 and 2, tests may be carried out using a 7-well series extending transversely of the plate, also called a "cassette". In most test procedures, a 7-well channel will be used, the first and seventh wells being control wells, and the other five wells for the serial dilutions. More particularly, a complete procedure can involve the following steps:

(1)    Isolate colonies.

(2)   With loop remove colonies and suspend in (37°C) enriched Brain Hear Infusion (sterile) broth, which may contain growth promutant additives.   (6 ml) six milliliters.

(3)   Adjust to 0.5 McFarland standard yielding suspension of $1.5 \times 10^8$ bacteria/ml.

(4)   Incubtate 37°C.

(5)   Prepare antibiotics solutions 1000 g/ml in saline.

(6)   Dispense 0.1 milliliter antibiotic into well #6 diluting serially 1:9 through well #2 in sterile plastic plate.

(7)   Dispense 0.1 milliliter sterile saline in wells #1 and #7:   dispense second 0.1 milliliter saline in well #1.

(8)   Perform similarly for eight (8) appropriate different antibiotics.

(9)   Dispense 0.8 milliliter of bacterial suspension (pure culture) into well #2 through #7.

The distribution in the spherical wells 1 to 7 is summarized below in Table A.   The fill volumes of 1.0 ml per well are designed to fill each well to the level of the bottom of the neck portions.

TABLE A

| Well Number | 1 | 2 | 3 | 4 | 5 | 6 | 7 |
|---|---|---|---|---|---|---|---|

| | Vol (ml) | Conc/ml | Vol (ml) | Conc/ml | Vol (ml) | Conc/ml | Vol (ml) | Conc/ml | Vol (ml) | Conc/ml | Vol (ml) | Conc/ml | Vol (ml) | Conc/ml |
|---|---|---|---|---|---|---|---|---|---|---|---|---|---|---|
| Antibiotic | 0.1 | (saline) 0 | 0.1 | 0.1/ml | 0.1 | 1. /ml | 0.1 | 10 /ml | 0.1 | 100 /ml | 0.1 | 1000 ml | 0.1 | (saline) 0 |
| Enriched Brain Heart Infusion Broth | 0.8 | -- | 0.8 | -- | 0.8 | -- | 0.8 | -- | 0.8 | -- | 0.8 | -- | 0.8 | -- |
| Bacteria | 0.1 | (saline) 0 | 0.1 | $1.5 \times 10^8$ | 0.1 | $1.5 \times 10^8$ | 0.1 | $1.5 \times 10^8$ | 0.1 | $1.5 \times 10^8$ | 0.1 | $1.5 \times 10^8$ | 0.1 | $1.5 \times 10^8$ |
| TOTAL | 1.0 | 0.0 µg 0 bacteria | 1.0 | 0.01 µg $1.5 \times 10^7$ bacteria | 1.0 | 0.1 µg $1.5 \times 10^7$ bacteria | 1.0 | 1.0 µg $1.5 \times 10^7$ bacteria | 1.0 | 10 µg $1.5 \times 10^7$ bacteria | 1.0 | 100 µg $1.5 \times 10^7$ bacteria | 1.0 | 0.0 µg $1.5 \times 10^7$ bacteria |
| | | Negative Control | | | | | | | | | | | | Positive Control |

13

The steps of reading, calculation, plotting, and interpretation of the data are summarized below.

READING:

With individual or ganged oxygen electrodes measure and record all $pO_2$ values 20 to 40 minutes following original inoculation of bacteria, the time being selected to be just prior to the beginning of logarithmic growth in the non-inhibited samples. All reactants are kept at 37°C during the procedure.

CALCULATIONS:

(a) $pO_2$ negative control - $pO_2$ positive control = span.

(b) $\dfrac{pO_2 \text{ observed (each antibiotic containing well)}}{\text{span}} \times = \% \ pO_2$

PLOT DATA: $pO_2$ concentration (% $O_2$ consumed) ordinate antibiotics concentration abcissa.

INTERPRETATION OF DATA:

$pO_2$ values which are equal to, or approach the positive control (near zero $pO_2$) demonstrate $O_2$ consumption indicating approach of logarithm growth and therefore NO antibiotic susceptibility.

$pO_2$ values which approach or equal those of negative control (near 100% $pO_2$) indicate virtually total inhibition of growth by antibictic.

$pO_2$ values intermediate between these values indicate partial sensitivity to the antibiotic due to lack of optimum concentration of antibiotic or resistance of organism.

The ideal antibiotic concentration is the first concentration to produce inhibition of oxygen consumption where the value of $pO_2$ observed approaches the value of the negative control.

## SINGLE CONCENTRATION ANTIBIOTIC SUSCEPTIBILITY TESTING

Up to twelve antibiotics can be tested utilizing two channels of the cassette (14 wells).

In this procedure, known optimum concentrations of antibiotic are dispensed. Where two (2) known antibiotic concentrations are known for different groups of organisms, two levels are dispensed. This is similar to the Kirby-Bauer procedure. The end points are the degree of inhibition of oxygen consumption.

PROCEDURE:

(1)  Isolate colonies.

(2)  With loop remove colonies and suspend in (37°C) enriched Brain Heart Infusion Broth (3 milliliters).

(3)  Adjust to 0.5 McFarland Standard yielding suspension of $1.5 \times 10^8$ bacteria/ml. Incubate 37°C.

(4)  Preprepare antibiotics solutions so that the optimum concentration/ml will be contained in 0.1 milliliter.

(5)  Dispense individual antibiotics solutions 0.1 milliliter in each designated well (#2 through #13). Dispense 0.1 milliliter sterile saline in wells #1 and #14.

0128527

(6)   Dispense 0.8 milliliter enriched Brain Heart Infusion broth into all 14 wells.

(7)   Inoculate wells #2 through #14 with 0.1 milliliter of bacterial suspension yielding concentrations of $1.5 \times 10^7$ bacteria/ml.   Add 0.1 milliliter saline to well #1.   Cover with plastic film and incubate 37°C.

(8)   After 30 minutes following initial inoculation measure $pO_2$ in all wells and record.

0128527

In addition to the use of an oxygen electrode as described herein, there are a number of other known methods for measuring oxygen in solution. These include:

(1) A variety of dyes are available for measurement of oxygen in solution. The dye is introduced in the reduced form. Oxidation of the dye produces a chromophore and hence, a change in color. Colorimetry can then be performed at the appropriate spectral wave length in order to determine oxygen concentration.

(2) A variety of spectroscopic methods are available to measure oxygen concentration directly.

(3) Oxygen concentration can be measured by electron spin resonance. A variety of other techniques are available for the measurement of oxygen.

By determining the antimicrobial susceptibility of oxiphilic bacteria with respect to a number of different antimicrobial agents, information can be obtained for use in classifying or identifying the particular bacteria. More specifically, the resistance patterns of individual bacterial species against clinically used antibiotics, non-clinically used antibiotics and other antibacterial compounds permit the formation of a data base wherein a pattern of resistance and sensitivity can be found to be unique to each species. This permits the antibiotic sensitivity method to be utilized as a high speed identification system for bacteria.

CLAIMS

0128527

I claim:

1. A method for accelerated determination of antimicrobial susceptibility of oxiphilic bacteria, comprising:

(a) obtaining a pure culture of the oxiphilic bacterium to be tested;

(b) preparing a series of sterile samples of an aqueous culture medium formulated to promote rapid growth of said test bacterium including at least one positive control sample, said samples being of corresponding volume and composition and having a known concentration of dissolved oxygen;

(c) incorporating in said samples other than control samples predetermined concentrations of a selected water-soluble antimicrobial to form a sample series of progressive antimicrobial dilution;

(d) inoculating said samples other than negative control samples with a predetermined quantity of said culture to obtain a selected cell concentration therein;

(e) culturing said samples under conditions favorable to the rapid growth of the inoculated bacterium in said positive control sample, said culturing being continued to a time in the latter part of the lag phase or the early part of logarithimic growth phase of the bacterium with reference to said positive control sample; and

(f) determining the extent of change in the dissolved oxygen concentration in the liquid phase of the samples as a measure of the susceptibility of the inoculated bacterium to the test antimicrobial, oxygen reduction in the medium correlating with antimicrobial resistance and/or antimicrobial concentration below a growth inhibitory level.

2. The method of claim 1 in which the extent of change of the dissolved oxygen concentration of said samples is determined by measuring the oxygen partial pressures with an oxygen electrode.

3. The method of claim 1 in which the culturing is terminated and the oxygen concentrations are determined during the transitional period comprising the end of the lag phase and the beginning of the growth phase.

4. A method for accelerated determination of antimicrobial susceptibility of oxiphilic bacteria, comprising:

(a) obtaining a pure culture of the oxiphilic bacterium to be tested;

(b) preparing a series of sterile samples of an aqueous culture medium formulated to promote rapid growth of said test bacterium including at least one positive control sample, said samples having a volume of from 0.5 to 1.5 milliliters (ml) and having a known concentration of dissolved oxygen;

(c) incorporating in said samples other than negative control samples predetermined concentrations of a selected water-soluble antimicrobial to form a sample series of progressive antimicrobial dilution;

(d) inoculating said samples other than negative control samples with a predetermined quantity of said culture to obtain a selected cell concentration in the range of about $10^6$ to $10^8$ cells/ml;

(e) culturing said samples under conditions favorable to the rapid growth of the inoculated bacterium in said positive control sample, said culturing being continued to the transitional period comprising the end of the lag phase and the beginning of the growth phase with reference to said positive control sample; and

(f) determining the extent of change in the dissolved oxygen concentration in the liquid phase of the samples as a measure of the susceptibility of the inoculated bacterium to the test antimicrobial, oxygen reduction in the medium correlating with antimicrobial resistance and/or antimicrobial concentration below a growth inhibitory level.

5. The method of claim 4 in which the extent of change of the dissolved oxygen concentration of said samples is determined by measuring the oxygen partial pressures with an oxygen electrode.

6. The method of claim 4 in which said samples are contained in test wells having generally spherical walls.

7. A method for accelerated determination of antibiotic susceptability of oxiphilic bacteria, comprising:

(a) obtaining a pure culture of the oxiphilic bacterium to be tested;

(b) preparing a series of sterile samples of an aqueous culture medium formulated to promote rapid growth of said test bacterium including at least one positive control sample, said samples having a volume of from 0.5 to 1.5 milliliters (ml), being of corresponding volume and composition, and having a known concentration of dissolved oxygen, said samples being contained in test wells having generally spherical walls;

(c) incorporating in said samples other than control samples predeterined concentrations of a selected water-soluble antibiotic to form a sample series of progressive antibiotic dilution;

(d) inoculating said samples other than negative control samples with a predetermined quantity of said culture to obtain a selected cell concentration in the range of about $10^6$ to $10^8$ cells/ml;

(e) culturing said samples under conditions favorable to the rapid growth of the inoculated bacterium in said positive control sample, said culturing being continued to the transitional period comprising the end of the lag phase and the beginning of the growth phase for the uninhibited control; and

(f) determining the extent of change in the dissolved oxygen concentration in the liquid phase of the samples as a measure of the susceptability of the inoculated bacterium to the test antibiotic, oxygen reduction in the medium correlating with antibiotic resistance and/or antibiotic concentration below a growth inhibiting level.

8. A microdilution plate for determining antimicrobial susceptibility of bacteria by oxygen depletion measurement, comprising a unitary plate assembley formed from two superposed mating plate sections of transparent plastic, the upper section providing a plurality of tranversely and longitudinally aligned access ports each communicating with a tubular downwardly-extending neck which connects to a hemispherical recess of enlarged diameter relative to said port and neck, the lower section providing complementary hemispherical recesses, said mating recesses providing generally spherical cavities for receiving the oxygen-containing cultures.

0128527

FIG. 1

FIG. 1A

FIG. 2

OXYGEN ELECTRODE

FIG. 3

PO₂ CONCENTRATION

ESCHERICHIA COLI
AND
CHLOROMYCETIN

MIC

0.00079μg    0.0079μg    0.079μg    0.79μg    7.9μg

ANTIBIOTIC CONCENTRATION μg/m